# EUROPEAN PATENT APPLICATION

(11) **EP 2 420 243 A1**
(43) Date of publication of application: **22.02.2012**
(21) Application number: 10173214.7
(22) Date of filing: 18.08.2010
(51) Int. Cl.: A61K 36/185, B01D 61/14, A61P 7/06

(54) **Compositions obtainable from bred beetroot juice to promote iron absorption and blood forming**

(71) Applicant: Inovativo Biomedicinas Tehnologiju Instituts, SIA, 1010 Riga (LV)
(72) Inventor: Babarikins, Dmitrijs, LV-1010, Riga (LV); Bêrzina, Nadezda M., LV-1035, Riga (LV); Aniscenko, Aleksandrs, LV-1057, Riga (LV); Krûmina, Guntra, LV-2135, Ropazu novads (LV); Babarikina, Anna, LV-1010, Riga (LV)
(74) Representative: Fortuna, Aleksandra

(57) **Abstract**

An invention pertains to manufacturing of medicines, food and food supplements and particularly products containing components facilitating iron absorption and haematopoiesis. The composition described is obtained by extraction of a complex of biologically active substances having molecular weight up to 10 000 Da from the red beetroot (*Beta vulgaris*) juice. The composition stimulates intestinal iron absorption better than the native red beetroot juice, ascorbic acid, citric acid or other commercially available medicines for facilitation of iron absorption. The composition may be used with other substances improving iron absorption in humans and animals.

## Description

### Technical field

Invention pertains to production of medicines, food and food supplements and particularly products containing components facilitating iron intestinal absorption and stimulating hematopoiesis.

### Prior art

Iron deficiency anemia (IDA) causes weakness, depression, impairment of functioning of all organs and systems as well as exacerbation of chronic diseases. These pathological conditions are caused by decreased iron reserve in human body, which in turn inhibits synthesis of hemoglobin.

In Asian countries IDA is diagnosed in 60% of reproductive age women and in 40-50% of pre-school age children. (J. M. Hurt. Reversing productivity losses from iron deficiency: the economic case. J. Nutr., 2002, 132, 794S-801S.). In the USA the IDA is one of the most common nutrition insufficiencies which causes premature delivery in 3.3 million women and retards development of newborns (B. L. Callen. Program of care for young women with iron deficiency anemia: a pilot. J. Community Health Nurs., 2000, 17, 4, 247-262.).

Likelihood of development of IDA is determined by relatively low level of absorption of iron in normal conditions (only up to 10% of totally ingested iron) and relatively wide range of absorption inhibiting factors (alimentary iron deficiency, intestinal and endocrinal diseases, bleeding, etc.). This makes actual the elaboration of relevant medicines and parapharmaceutical products.

Iron absorption from commercially available pharmaceutical formulations is weak and the medicines should be used in high doses. Daily dose of preparation *Ferroplex* (contains 30 mg ascorbic acid, 50 mg iron sulphate which corresponds to 10 mg of elemental iron and excipients - gelatin, stearin, starch, *gummi Acaciae,* vegetable oil, magnesium, talc, lactose, sucrose) is up to 6 tablets. Common undesirable side effects of iron containing medicines are nausea or vomiting (D. Wyck et al. A randomized, controlled trial comparing IV iron sucrose to oral iron in anemic patients with nondialysis-dependent CKD. Kidney Int., 2005, 68, 2846-2856).

There are two approaches to the development of medicines for effective treatment of IDA: attempt to find different new iron compounds or to use iron ions absorption facilitating substances. Currently the following iron compounds for manufacturing medicines are in use: iron chloride tetrahydrate (Hemofer, Medana Pharma Terpol, Poland); iron hydroxide (Ferric(III)-hydroxyde polyisomaltose complex; Ferrum Lek, Lek Pharmaceuticals, Slovenia); iron sulphate (Acidum ascorbicum, Ferrosi sulfas; Ferroplex, Teva Pharmaceuticals, Hungary) and others. Bivalent iron compounds are used more commonly because iron penetrates intestinal cell's membrane and flows into blood circulation in the form of bivalent ions. In order to increase medicine's efficacy several iron compounds are combined and different usage schemes are applied in order to prevent oversaturation of intestinal mucosa by iron ions, inhibiting transport processes (patent application WO 2006/068697 A2, publ. date 29.06.2006). However the medicines containing iron compounds do not stimulate active transport of Fe⁺² through intestinal mucosa, but stimulate passive process which is not manageable and depends on iron ions content of intestines. As a result the body supply of iron is governed by a range of risk factors.

As iron ions absorption facilitators the following substances are included in medicines and food: imidazole derivatives (patent JP 7097323, publ. date 11.04.1995), organic acids (patent application WO 2006/068729 A2, publ. date 29.06.2006) including vinegar produced from different raw materials (patent JP 2003146873, publ. date 21.05.2003), however the most commonly used substance is ascorbic acid (M. Hoppe et al. The relative bioavailability in humans of elemental iron powders for use in food fortification. Eur. J. Nutr., 2006, 45, 1, 37-44; patents EP 0397232, publ. date 22.03.1995.).

In recent years studies are conducted with the aim to find new natural substances facilitating absorption of iron. Mexican patent application (MX NL01000021A, publ. date 28.02.2002) suggests the use of animal blood components, other studies (patent CN1451395, publ. date 29.10.2003) suggest the following: the use of cartilage tissue or chondroitin sulphate combined with zinc compound (patent application US20060177516A1, publ. date 10.08.2006), casein phosphate peptides (patent JP59162843, publ. date 13.09.1984). For the same purpose herbal components may be used. Japanese authors suggest to use components of tea leaves (JP 2001139481, publ. date 22.05.2001) or fermented guarana gum and seeds (JP6247860, publ. date 06.09.1994). Chinese traditional medicines usually are multi-component. The composition containing *Polygonatum sibiricum, Salvia officinalis, Dolichoris album* seeds, *Atractylodes lancea, Schisandra chinensis* and hawthorn fruits is also known (CN1110172, publ. date 18.10.1995).

Powder of hawthorn fruits and carrots enriched by iron and vitamin C in combination with folic acid is used in traditional medicinal treatment of anemia (CN1348785, publ. date 15.05.2002). A fungus *Ganoderma lucidum* in combination with 8 other components is used for the same indications (patent CN1634535, publ. date 06.07.2005).

It is known that carbohydrates may affect the absorption of Fe³⁺ ions in intestines (Charley et al. Chelation of iron by sugars. Biochem. Biophys. Acta, 1963, 69, 313-321). The use of complexes of iron and carbohydrates, especially complexes of iron and mono- and di-polysaccharides, for treatment of iron deficiency is described in patent application WO 2007/081744 A2 (publ. date 19.07.2007). The product is for oral and parenteral administration. Authors emphasize the safety of the composition which is higher than that of the widely used pharmaceutical substances like iron dextran and iron saccharate.

Traditional medicine for discontinuation of vaginal bleeding and treatment of anemia suggests the use of beetroot juice as a source of iron (A. Fugh-Berman et al. Treatment of fibroids: the use of beets (Beta vulgaris) and molasses (Saccharum officinarum) as a herbal therapy by Dominican healers in New York City. J. Etnopharmacol., 2004, 92, 337-339.). Beetroot juice decreases blood pressure in high doses (500 ml) and provides protective effect on blood vessels. These effects are associated with bioconversion of juice nitrates into nitrites (Webb A.J. et al. Acute blood pressure lowering, vasoprotective and antiplatelet properties of dietary nitrate via bioconversion to nitrite. Hypertension, 2008, 51, 3, 784-790).

Recently beetroots have become an object of extensive studies due to their potential to be a source of industrial natural pigments and for their anticancerogenic properties (G. J. Kapadia et. al. Chemoprevention of DMBA-induced UV-B promotes, NOR-1-induced TPA promoted skin carcinogenesis, and DEN-induced phenorabital promotes liver tumors in mice by extract of beetroot. Pharmacol. Res., 2003, 47, 2, 141-148.).

Currently the extraction technology of biologically active substances from beetroots has been developed for the purpose of production of pigments. These pigments are used as a natural dyestuff mostly in food industry and for prevention and treating purposes as an antioxidant composition (patent application US 2003/0198694 A1, publ. date 23.10.2003).

The technology of beetroot pigments manufacturing (fractioning) is based on water extraction method which involves at least two drying steps: firstly, the drying of juice followed by drying (concentration) of product obtained (patent CA 1171717, publ. date 31.07.1984; patent application US 2003/0036565A1, publ. date 20.02.2003), however the technology involves a high consumption of energy.

Other technological processes are concerned with increase of pigment output: the juice is fermented to be purified from sugar (patent FR 2399467, publ. date 02.03.1979) or processed with pectolytic and cellulolytic enzymes (WO 98/26792, publ. date 25.06.1998). Some authors describe specific technologies used in production of beetroot juice for human consumption. For ensuring the product with natural taste, color and stability the lemon juice is used (patent application W02008098273, publ. date 21.08.2008). There are other methods known for increasing the output of juice (patent application CN101433349, publ. date 20.05.2009).

### Disclosure of invention

The objective of this invention is a composition for facilitating intestinal absorption of iron ions and stimulation of hematopoiesis in bone marrow, comprising beetroot, preferably red beetroot (*Beta vulgaris var. Esculenta f. Rubra DC*) or its components including the juice. The composition may additionally comprise iron compounds, ascorbic acid and citric acid.

Experimentally we disclosed that *Beta vulgaris,* especially red beetroot and its juice is not only a source of iron, but also comprise substances specifically facilitating iron absorption; they enhance absorption of iron introduced *per os,* as well as iron ions, already present in digestive tract.

Results of the studies performed by inventors substantiate conclusion that the red beetroots not only facilitate absorption of iron and synthesis of hemoglobin but affects also blood cells proliferation.

The invention provides the composition stimulating absorption of iron and hematopoiesis obtainable by fractioning juice of *Beta vulgaris,* preferably *Beta vulgaris var. Esculenta f. Rubra DC,* resulting in production of biologically active composition of substances - red pigment and complexes of other juice components, having the molecular weight up to 10 000 Da, preferably up to 5 000 Da.

It is disclosed and experimentally proved that the capability to stimulate the absorption of iron ions in intestinal wall by the complex of components of *Beta vulgaris* juice produced in this way is higher than that of native red beetroot juice. It is experimentally proved that the combination of the complex of juice components of *Beta vulgaris* as described here with iron, ascorbic acid and citric acid is more active than the commercially available most widespread used preparations (e.g., Ferroplex, "Teva Pharmaceuticals", Hungary; Hamatopam 100, "Biotest Pharma", Germany) where the active substances are iron salts and ascorbic acid.

### Brief description of figures

Fig. 1 represents the graph of iron accumulation in the mucosal cells of duodenum in chicken after oral administration of red beetroot juice of various concentrations or of fraction II of the juice fractioned by ultrafiltration.
Fig. 2 represents the graph of iron accumulation in liver after oral administration of red beetroot juice of various concentrations or of juice fraction II prepared by ultrafiltration.
Fig. 3 represents dynamics of blood hemoglobin concentration in rats undergone experimental acute blood loss after oral administration of red beetroot juice or the fractioned juice.

In *in vitro* experiments using segments of chicken's small intestine ("mucosa accumulating preparation" or MAP) as developed by A.Ugolev and co-authors (Y 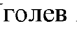 A.M. 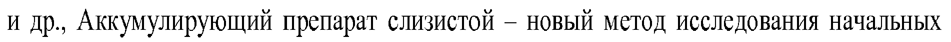 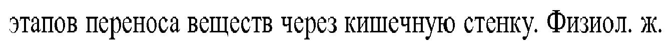 CCCP, 1970, 56, 11, 1638-1641.) it was found, that the juice of red beetroot has the specific iron absorption facilitating activity in comparison with juice of other vegetables (Table 1).

**Table 1: Effect of vegetable juice on iron absorption in small intestine mucosa in chicken (Lohmann Brown) in vitro***

| **Source of juice** | **Iron concentration in intestinal mucosa (mkg/g) after incubation for 30 minutes at 40°C *in vitro*** | |
|---|---|---|
| | ***Duodenum*** | ***Jejunum*** |
| **1. Summer squash** | 9.80±0.58*** | 3.84±0.22* |
| **2. Carrots** | 9.15±0.69*** | 5.14±0.72** |
| **3. Cucumbers** | 8.15±0.29* | 6.89±0.42**** |
| **4. Pumpkins** | 8.12±0.38*** | 5.12±0.37* |
| **5. Red beetroots** | 11.85±0.80 | 10.38±1.46 |

| | | |
|---|---|---|
| * in all measured samples of juice the iron and of ascorbic acid concentration was normalized up to 30 mkg/ml and 0.05 mM respectively; n=20. Statistical significance of difference comparing to data from red beetroot juice: *p<0.001; **p<0.002; ***p<0.003****p<0.02. | | |

The fractioning of the juice was performed in order to increase the specific activity of red beetroot juice. The technology was developed by using the red beetroots grown in district of Aizkraukle in vicinity of Sk 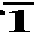 rveri (Latvia) and harvested in September 2009.

The beetroots were shred and the juice was extracted mechanically by juice press. The fractioning was performed by gel-filtration method using Sephadex G-25 coarse. Columns (18.0 x 2.4 cm) were balanced with distilled water. Cellular gel was prepared from polysaccharide dextran Sephadex which was used for separation of mixtures containing components of different molecular weight. Each Sephadex type allows to separate biomolecular particles of different size according to their molecular weight: G-25 (1000-5000 Da), G-50 (1500-30000 Da), G-75 (3000-70000 Da).

The juice obtained was heated in water bath at 90°C for 10 minutes, then centrifugated at 5000g for 15 minutes and 10 ml of the juice was put into column. Eluation was performed with distilled water at flow speed of 96 ml/h using peristaltic pump. As a result two fractions were obtained: I ― in 28 ml and II ― in 50 ml volume. The second fraction was dried by rotating vacuum dryer at 40°C (1 kPa).

Optimal fractioning regime was established based on the results of laboratory investigations. Sephadex G-25 ensured faster separation of high molecular proteins with minimal volume of eluate comparing to G-50. By using G-75 3 peaks (fractions) were obtained, however the fraction with maximal specific activity was distributed between peaks - I and III. Volume of fractions obtained by gel-filtration on Sephadex G-25 was 3 times less than that obtained by G-50. This made easier concentration of biologically active substances. Increase of ratio of sample slice height to length of column from 1:8 8 to 1:10 provided better fractioning of red beetroot juice, however further increase to 1:11 did not affect the results substantially. Increase of eluation speed above 96 ml/min caused delay of fractioning, but slower eluation caused undesirable increase of the volume of fraction II.

In order to evaluate the specific activity of the vacuum-dried juice fraction II, the obtained composition was diluted up to 10 ml by distilled water, because this quantity of the fraction was obtained from 10 ml of juice.

Examination of the effect of red beetroot juice fraction II on the iron absorption was performed *in vitro, in situ* and *in vivo* on chicken and rat.

Regimes of heating and centrifugating steps of the technology for producing active fraction of red beetroot juice were chosen based on specific activity of the fraction II of red beetroot juice study results *in vitro* (Table 2).

**Table 2: The effect of some parameters of red beetroot juice fractioning technology on the capability of the composition obtained to stimulate iron absorption in chicken intestinal (jejunum) everted segments in vitro.**

| Details of the method see in Table 1. | | |
|---|---|---|
| **Technical parameter** | **Value of the parameter** | **Iron concentration in mucosa (mkg/g) of intestinal segments after 30 min incubation at 40°C** |
| **Time of heating, min** | 5 | 12.14+0.16 |
| | **10** | **13.44+0.94** |
| | 15 | 13.22+0.75 |
| | 20 | 10.81+1.03 |
| **Heating temperature Centigrade's** | 70 | 10.73+1.12 |
| | 80 | 11.86+0.74 |
| | **90** | **13.07+0.67** |
| | 100 | 12.01+0.25 |
| **Time of centrifugation, min** | 10 | 12.00+0.36 |
| | **15** | **13.03+0.44** |
| | 20 | 12.02+0.40 |
| **Speed of centrifugation, g** | 4700 | 13.12+0.33 |
| | **5000** | **13.20+0.12** |
| | 5300 | 10.84+0.76 |

Gel-filtration is an appropriate fractioning method for separation of substances mostly on laboratory scale because the process is labor and material consuming and expensive. For this reason the fractioning of red beetroot juice on industrial scale was performed by more appropriate filtration method.

Filtration is widely used method for fractioning liquids. According to capability to separate components the filtration methods are classified as follows: clarification up to 50 µm; microfiltration 50 µm - 0.1 µm; ultrafiltration 0.1 µm - 0.001 µm; reverse osmosis <0.001 µm. (http://www.wwdmag.com/Filtering-Through-the-Claims-of-Varying-Water-Treatment-Methods-article 668).

The technological problem to solve was the change gel-filtration to fractioning method using semi-permeable membrane. Ultrafiltration through filters having appropriate pores size may be relatively easy to use for fractioning single or some components solutions for obtaining product with desirable molecular weight. According to theoretical essentials of ultrafiltration it is difficult to predict the outcome of ultrafiltration of multi-component solutions (especially such as plant juices) because the process is affected by very many factors: molecular weight of components, concentration, solution's viscosity, temperature, hydrostatic pressure, flow speed, etc. Therefore it is possible to elaborate practically useful ultrafiltration technology for obtaining the product similar to the product obtainable by gelfiltration by experimenting with different ultrafilters, changing process parameters and testing biological specific activity of the prepared compound's compositions.

Use of membrane technology essentially optimizes the step of juice preparation for the following fractioning: centrifugation is replaced by microfiltration, which dramatically increased productivity of the technology. The filtration was performed using "Amafiltergroup" bag filter in the following stages: 250 µm filter, 50 µm filter, filtrate was heated for 10 min at 90°C , 250 µm filter, 50 µm filter, 5 µm filter, 1 µm filter.

Additional experiment was performed in order to find the optimal parameters for the last stage of filtration more precisely (Table 3). In last stage the product was filtered through "Amafiltergroup" microfilters having pore size 1.0 and 0.5 µm and the speed of the process was measured.

**Table 3: Speed of product filtration using "Amafiltergroup" microfiltration bags with different pore sizes.**

| **Technical parameter** | **Filter pore size** | **Filtration speed at the 1st stage of ultrafiltration,1/h** |
|---|---|---|
| **Bag microfilters, µm** | 1.0 | 2.1±0.3 |
| | 0.5 | 1.9±0.2 |

The filter having pore size 1.0 µm was found the most cost effective, because the decrease of pore size to 0.5µm did not increase the speed of ultrafiltration while increased the expenses.

In order to find optimal ultrafilter which allows to produce the product having biological activity similar to that obtained by gel-filtration the inventors experimented with several capillary ultrafilters. Specific biological activity of the red beetroot juice permeate obtained by ultrafiltration was evaluated on chickens *in vivo* after oral administration of the single dose of the product. Duodenum and liver were extirpated 90 minutes after administration of fractioned red beetroot juice (Table 4).

**Table 4: Specific biological activity (in vitro) of fractioned red beetroot juice dependence on parameters of the ultrafilter used for preparing the product .**

| **Characteristics and methods of use of the ultrafilters for fractioning red beetroot juice** | **Iron content in duodenum mucosa (mkg/g)** | **Iron content in liver (mkg/g)** | **Evaluation of efficiency of ultrafiltration process** (acceptable minimal speed was 10 ml/min) |
|---|---|---|---|
| UF-1 Capillary composite polysulfonic membrane with ultrafiltration coefficient 68 mL/h*mmHg | 16.4± 0.9 | 89.1 ±2.5 | Product after heating and filtrating through filter UF-1. Speed of filtration process is satisfactory (25 ml/min) |
| UF-2 Capillary composite polysulfonic membrane with ultrafiltration coefficient 13 mL/h*mmHg | Not tested | Not tested | Product after heating and filtrating through filter UF-2. Process stopped after 2 minutes |
| UF-3 Capillary composite polysulfonic membrane with ultrafiltration coefficient 10 mL/h*mmHg | Not tested | Not tested | Product after heating and filtrating through filter UF-2. Process stopped after 1-2 minutes |
| UF-4 Capillary composite polysulfonic membrane with ultrafiltration coefficient 6,8 mL/h*mmHg | Not tested | Not tested | Filtration of the product after heating and filtrating through filter UF-2 was insufficient. Process stopped after 2-3 minutes |
| UF-1 → UF-2 | 22.4 ±0,7 | 94.6 ±3.0 | Product filtered through both filters with sufficient speed (20 ml/min) |
| UF-1 → UF-3 | 18.7± 0.9 | 96.5 ±1.9 | Process speed was satisfactory (20 ml/min) |
| **UF-1 → UF-4** | **21.2**±**1.0** | **113.5**± **2.3** | **Process speed was satisfactory (15 ml/min)** |
| UF-1 → UF-3 → UF-4 | 20.3 ±0.6 | 103.0± 2.1 | Process speed was 10 ml/min |
| Fraction II of red beetroot juice obtained by gel-filtration | 21.3± 0.5 | 111.8 ±1.8 | Speed is slow and not comparable with capillary ultrafilters |

Conclusions drawn from the results of the experiment with various regimes of ultrafiltration (Table 4) are that in spite of slower ultrafiltration speed the preferable technology should be version UF- 1 → UF-4. Under these conditions the product obtained has the lower capability to facilitate absorption of Fe⁺² in intestinal mucosa, while according to iron accumulation in liver the product has highest overall biological activity. Iron accumulation in the liver is an integral index and it characterizes ions intestinal transport not only at the initial stage (on intestinal mucosa level), but the process in total and it corresponds more closely to the results of the fractioned red beetroot juice, produced by gel-filtration.

To further increase the efficiency of the ultrafiltration process filters analog to UF-1 and UF-4 made of other polymers characterized by higher ultrafiltration coefficient were evaluated. The red beetroot juice and fractionated one were tested for their biological activity *in vivo* and some substances, which are crucial for the specific effect were determined as well. At the same time other ultrafilters were estimated as described below.

**Table 5: In vivo dependence of specific biological effect of fractioned red beetroot juice on parameters of the ultrafilter used for preparation of the product.**

| **Name** | **Beta-cyanins mg/100 ml** | **Beta-xanthines mg/100 ml** | **Betaine ,%** | **Iron content in duodenal mucosa 90 min after admin. in vivo, mkg/g** | **Iron content in liver 90 min after admin. in vivo, mkg/g** | **Sucrose %** | **Glucose, %** | **Fructose, %** | **Lysozyme mkg/ ml** |
|---|---|---|---|---|---|---|---|---|---|
| Red beetroot juice | 59.6± 1.2 | 34.4±1.0 | 0.296± 0.009 | 23.4±5.00 | 90.42± 12.08 | 9.96± 0.81 | 0.89± 0.09 | 0.76 0.07 | 10.5 ±0.5 |
| **UF-1*** | 55.0± 2.1 | 33.7±1.3 | 0.669± 0.018 | 25.9±2.73 | 94.58± 12.27 | 11.37± 0.40 | 1.16± 0.06 | 0.86 0.06 | 12.5 ±0.3 |
| **UF-1**→**UF-4*** | 29.2± 0.9 | 20.4±0.9 | 0.245± 0.007 | 19.7±2.89 | 102.5± 13.52 | 9.50± 1.04 | 0.98± 0.07 | 0.60 0.07 | 5.0± 0.4 |
| **UF-5**** | 57.8± 1.1 | 34.2±1.9 | 0.512± 0.015 | 20.1±3.45 | 87.58± 11.43 | 12.02± 6.58 | 1.09± 0.06 | 1.08 0.65 | 10.0 ±0.2 |
| **UF -5**→**UF-4** | 34.4± 1.5 | 21.9±1.7 | 0.252± 0.010 | 24.6±2.07 | 82.92± 5.66 | 8.88± 8.60 | 0.93± 0.11 | 0.77 0.03 | 6.5± 0.4 |
| **UF-5**→**UF-6 ***** | 46.9± 2.0 | 29.1±0.6 | 0.353± 0.014 | 27.1±3.53 | 94.58± 14.87 | 10.86± 6.48 | 1.09± 0.08 | 1.09 0.05 | 12.5 ±0.4 |
| **Fraction II of red beet-root juice obtained by gelfiltration** | 44.9± 1.3 | 28.5±1.7 | 0.112± 0.009 | 26.0±6.02 | 93.33± 13.29 | 18.14± 5.57 | 0.99± 0.08 | 0.23 0.09 | 12.5 ±0.3 |

| | | | | | | | | | |
|---|---|---|---|---|---|---|---|---|---|
| *Notes: See characteristics of UF-1 and UF-4 ultrafilters in Table 4. **UF-5 - Capillary composite poly(aryl-ether-sulfonic), polyvinylpirrolidone and polyamide membrane with ultrafiltration coefficient 70 mL/h*mmHg ***UF-6 - Capillary composite poly(aryl-ether-sulfonic), polyvinylpirrolidone and polyamide membrane with ultrafiltration coefficient 10 mL/h*mmHg | | | | | | | | | |

Examination of the effect of red beetroot juice fraction separated by ultrafiltration on the absorption of iron was performed *in situ* and *in vivo* with chicken and rat models.

In *in situ* study iron absorption in duodenum ligated loop (5 cm) was evaluated for 30 minutes. 0.5 ml of red beetroot juice or its fraction with added iron sulphate (400 mkg/ml iron) were introduced into the intestine. At the end of the experiment iron transportation in duodenal mucosa and liver accumulation was determined (Table 6).

**Table 6: Effect of red beetroot juice and its fraction on iron duodenal absorption and accumulation in liver in situ experiment.**

| **No** | **Experiment's design** | **Iron absorption in duodenum** | | **Iron accumulation in liver (mkg/g)** |
|---|---|---|---|---|
| | | **mkg/g** | **% of administered** | |
| **1.** | **Buffer solution* + iron ions** (400 mkg/mv)** | 32.92+2.50 | 15.3 | 144.25±9.11 |
| 2. | **Red beetroot juice + iron ions** (400 mkg/ml)** | 55.69±6,00 | 27.8 | 148.00±3.90 |
| 3. | **Red beetroot juice fraction + iron ions** (400mkg/ml)** | 138.19±9.06 p<0,001 (the 2nd v. the 3rd group) | 44.0 | 169.50±11.03 p<0.03(the 2nd v. the 3rd group) |

| | | | | |
|---|---|---|---|---|
| *Buffer solution composition: 13.7 mM Tris, 119 mM NaCl, 4.74 mM KCl, 20 mM fructose, pH 7.4. **Iron in a form of FeSO₄·7H₂O | | | | |

Table 6 shows that the fraction of red beetroot juice obtained by ultrafiltration statistically significantly more effectively stimulates iron transport in intestine and accumulation in liver.

Currently the most commonly used iron ions absorption facilitating agent in pharmaceutical and food industry is a vitamin C (ascorbic acid). This effect was studied in more details in previous *in vitro* and *in vivo* experiments (N.B 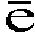 rzina et al., Influence of different dietary ascorbic acid levels and storage of iron on oxidative stability in chicken tissues. In: Advances and Challenges in Poultry Science. Proc. 1st Mediterranean Summit of WPSA, Greece, 2008) and the results obtained are presented in Table 7.

**Table 7: Effect of ascorbic acid on iron absorption in duodenum segment in chicken (Lohmann Brown) in vitro.**

| The *in vitro* experiment was performed by incubation for 30 minutes at 40°C. | | | |
|---|---|---|---|
| Version | Iron, mkg/ml | Ascorbic acid, mM | Accumulation of iron in duodenum mucosa, mkg/g |
| *Buffer solution + **iron | 28.6 | _ | 9.72±0.33 |
| *Buffer solution+**iron+asc orbic acid | 28.6 | 0.05 | 11.50±0.24 (p <0.05) |

| | | | |
|---|---|---|---|
| *Buffer solution composition: 13.7 mM Tris, 119 mM NaCl, 4.74 mM KCl, 20 mM fructose, pH 7.4. **Iron in a form of FeSO₄·7H₂O | | | |

As Table 7 shows the ascorbic acid at concentration of 0.05 mM increased the accumulation of iron in intestinal mucosa by 18.3%.

Effect of native red beetroot juice and its fraction on iron absorption in intact chicken were compared after administration of iron sulphate combined with ascorbinic acid. For this purpose the medicine Hamatopan 100 (Biotest Pharma, Germany) having the following content was used: 154.6 mg iron(II) sulphate 1 H₂O (corresponding to 50 mg of iron), 50 mg ascorbinic acid, excipients like calcium carbonate, carboxymethyl starch sodium (type A), carnauba wax, microcrystalline cellulose, powder cellulose, dibutylphtalate, ethylcellulose, gum Arabic, poly[butylmethacrylate-co-(2-dimethylaminoethyl) methacrylate-co-methylmethacrylate] (1:2:1), sucrose, shellac, high dispersion silica, talc, titanium dioxide (E171), tragant (E413), bleached wax) where the active substances were iron salts and ascorbinic acid.

Tablets were crushed in pestle and administered to chicken in a form of water suspension (in amount which contains 2 mg iron) or in combination with red beetroot juice or its fraction for 4 days. Iron accumulation in liver representing intensity of absorption of iron in intestine, as well as iron content in spleen (this organ acts as a filter of "aging" erythrocytes where iron accumulates) were determined. The results are presented in Table 8.

**Table 8: Effect of medicine Hamatopan 100 per se and of a combination with native and fractioned red beetroot juice on accumulation of iron in chicken liver and spleen.**

| **Study product** | **Iron content, mkg/g (n=7)** | |
|---|---|---|
| | **Liver** | **Spleen** |
| (Hamatopan 100) | 182.2 ± 11.2 | 120.0 ± 4.6 |
| 2 ml distilled water, | | |
| 2 mg iron Fe²⁺ | | |
| 2 mg ascorbic acid, | | |
| respective excipients | | |
| (Hamatopan 100+ red beetroot juice) | 173.0 ± 20.7 | 153.3 ± 3.8 |
| 1 ml distilled water, | | |
| 2 mg iron Fe²⁺ | | |
| 2 mg ascorbic acid, | | |
| 1 ml beetroot juice | | |
| **(Hamatopan.100+ red beetroot juice fraction)** | 206.7 ±1.89* | 165.2 ± 10.5** |
| 1 ml distilled water, | | |
| 2 mg iron Fe²⁺ | | |
| 2 mg ascorbic acid, | | |
| 1 ml beetroot juice fraction | | |

| | | |
|---|---|---|
| **Note:** iron and ascorbic acid were administered in a form of Hamatopan 100 (with its excipients) in the respective dose. Statistically significant difference comparing with the effect of Hamatopan 100: *p<0.02; **p<0.01 | | |

Table 8 shows that the red beetroot juice fraction statistically significantly more efficiently stimulates accumulation of iron in liver and spleen comparing to the effect of native juice and Hamatopan 100 .

In order to evaluate anti-anemic efficacy of red beetroot juice and its fraction the experiments were performed with Wistar-Kyoto rats with induced alimentary iron deficiency (ADD). The rats with ADD were obtained by feeding semi-synthetic ration according to the method of M.G.McCall and co-authors (Studies in iron metabolism. The experimental production of iron deficiency in the growing rat. Brit.J.Nutr., 1962, 16, 297-304). Within one month the iron deficiency anemia developed in animals showing explicit decrease of level of blood hemoglobin and body weight gain retardation. Within fourteen days the medicine Hamatopan 100 or its combination with the testing substances were administered *per os* in a single daily dose (in 1 ml of distilled water). The results are represented in Table 9:

**Table 9: Effect of native and fractioned red beetroot juice on blood parameters in rats having iron alimentary deficiency (IAD).**

| No. | **Group (n=7)** | **Hematocrit, %** | **Hemoglobin, g/dl** |
|---|---|---|---|
| 1. | **Norm** | 45.98± 0.92 | 15.71± 0.92 |
| 2 | **Control - IAD** | 32.55*** ± 1.87 | 10.10±*** 0.54 |
| 3 | **IAD + Fe + IAD+Fe+ascorbic acid** | 37.85* ± 2.03 | 12.85± * 1.05 |
| 4 | **IAD + Fe + IAD+Fe+ascorbic acid+ red beetroot juice** | 39.32* ± 1.49 | 13.17±** 1.04 |
| 5 | **IAD + Fe + ascorbic acid+ red beetroot juice fraction** | 45.77*** ± 1.99* | 15.02± ** 0.89 |

| | | | |
|---|---|---|---|
| Statistically significant difference comparing groups 1-2;2-3;2-4;2-5 and 1-5: *p<0.02; **p<0.01; ***p<0.001. **Note**: iron and ascorbic acid were administered to group 3-5 in a form of Hamatopan 100 (with the respective excipients) in the respective dose. | | | |

In order to examine whether the amount of carbohydrates and presence of pigment betalain in red beetroot juice affects iron absorption in intestines such varieties of *Beta vulgaris* without red pigment were examined: sugar-beets *(B.vulgaris var.Esculenta f.Altissima RÖSS)* and fodder beets *(B.vulgaris var.esculenta f.Lutens DC).* Analyzing the content of carbohydrates in red beetroot juice the following data were obtained: in fresh, centrifugated red beetroot juice the total amount of sugar was in the range of 3.0-7.4 %, amount of reducing sugars - 0.75-1.85%; in sugar-beets 14.0-17.5% and 1.98-2.55%; and in fodder beets - 8.0-11.7% and 1.12-2.20% respectively. According to the data of inventors in *vitro* activity of facilitation of iron absorption in sugar-beets *(B. vulgaris var. Escuelnta f. Altissima RÖSS)* is by 18% and in fodder beets *(B. Vulgaris var. Esculenta f. Lutens DC)* by 29% lower than in red beetroots.

The fractioned red beetroot juice has the profile of level of reducing sugars similar to the level in native juice and by 10-15% higher content of total sugar.

It is proved that carbohydrates (fructose, glucose, starch, etc.) reduce absorption of Fe²⁺ ions in intestines by lowering the concentration of soluble iron (Brouwer et al. Dietary fructose v. Glucose lowers ferrous-iron absorption in rats. Brit.J.Nutr. 1993, 70,171-178). Notwithstanding this fact the native red beetroot juice and its fraction stimulate iron absorption.

Fraction of the juice in comparison to the native juice contains less iron (3.13 and 1.5 mkg/ml accordingly) and ascorbic acid (14.4 mg% and 12.9 mg% accordingly). However the fraction of the juice can statistically significantly enhance the absorption of iron by 19-250% comparing to the native red beetroot juice depending on the compared index.

The data above allows to draw a conclusion that the higher specific activity of the fractioned red beetroot juice is a result of the effect of multi-component composition and not linked to the difference of contents of carbohydrates and pigments in both products. The most important components are: betacyanins, betaxanthines, betain, sucrose and lysozyme.

The addition of citric acid (300-380 mg/100 ml, preferably 320 mg/100 ml) to the fractioned red beetroot juice increasing the ability to facilitate iron absorption by 10-15% may be explained by improved bioavailability of the mineral element (Teucher B. et al.,Enhancers of iron absorption: ascorbic acid and other organic acids.Int.J.Vitam.Nutr.Res.,2004,74,6,403-419). It is important to mention that the citric acid at pH 4-6 stabilizes pigment betacyanin extending the expiry date of the product.

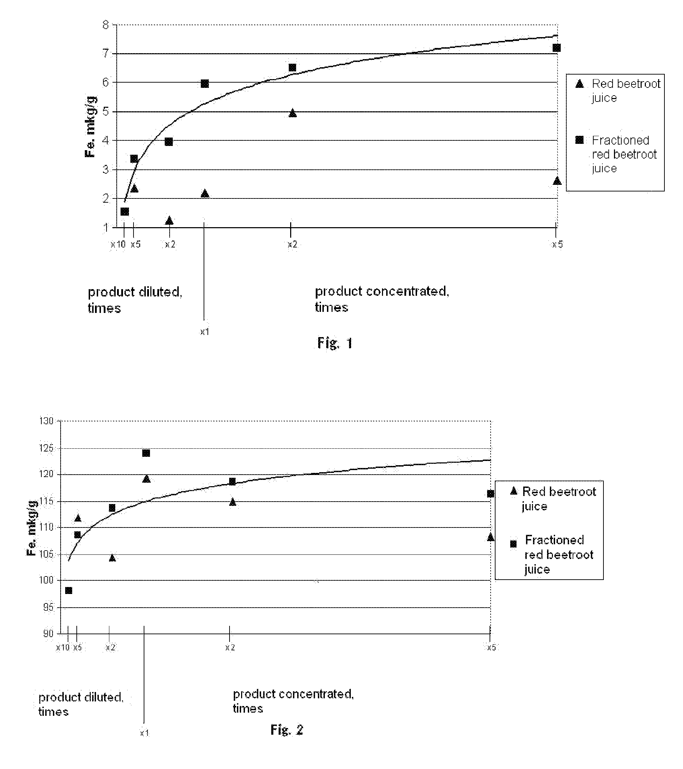

The iron transport facilitating activity of fractionated red beetroot juice is a dose-dependent. Specific effect of 2-5-10 times diluted or concentrated product evaluation results indicated that 2 times concentrated product shows better stimulating effect determined by Fe⁺² ions intestinal transport as well as accumulation of the element in liver (Fig. 1 and 2). Shape of the ,,dosc-effect" curve of fractioned red beetroot juice shows that the product, unlike native juice, enhances iron active transport, which is an energy-dependent physiological process. That means, that the product obtained can stimulate iron absorption in secondary iron deficiency cases, where as a result of some diseases the iron intestinal transportation mechanisms are disturbed.

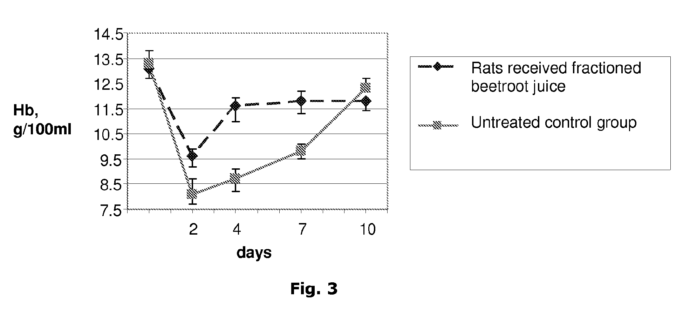

The optimal composition of the product stimulating iron absorption and hematopoiesis was found experimentally in rats with experimental acute blood loss model. The 4-4.5 ml of blood was drawn from sublingual vein in rodents having average body weight of 190 g followed by intravenous infusion of replacing saline. The rodents of control group did not receive any supplemental treatment during 10 days while the others received orally a daily dose of 1 ml of fractioned red beetroot juice supplemented with 15 mg/100 ml iron (in a form of bivalent iron compounds), 50 mg/100 ml ascorbic acid, 320 mg/100 ml citric acid and 100 mg/100 ml sodium benzoate and 50 mg/100 ml potassium sorbate as preservatives. The laboratory and biochemical blood investigation was performed at days 2-4-7-10. Dynamics of blood hemoglobin concentration is shown in Fig. 3. At the end of the experiment iron content in serum was 40.0 ±13.3 mkg/ml in control group and 74.7 ± 25.8 mkg/ml in group received the fractioned red beetroot juice (p=0.015). The dynamics of blood cells amount shows, that rodents treated by the fractioned juice restore blood parameters not only due to internal depot (in liver and spleen), but starting from the day 7 due to direct stimulation of hematopoiesis in bone marrow. The effect was more significant in female than in male rats.

The ability of the fractioned red beetroot juice to stimulate hematopoiesis in chicken model with experimental cadmium intoxication was examined also. Cadmium ability to induce anemia is well known. Pathogenesis of this effect is complicated, but the dominant role is played by renal tubules epithelium damage and inhibition of erythropoietin synthesis (a hormone inducting hemoglobin synthesis) (Horiguch; H. Anemia induced by Cadmium intoxication. Nippon Eiseigaku Zasshi., 2007, 62, 3, 888 - 4).

Lohmann Brown chicken of 35 days age received a standard diet for 10 days (1 g), diet with cadmium chloride (50 mg Cd mg/kg fodder) and a food supplemented by cadmium with simultaneous oral daily 1 ml dose of the fractioned red beetroot juice (enriched by iron, ascorbic acid as described above).

The results of the experiment are shown in Table 10.

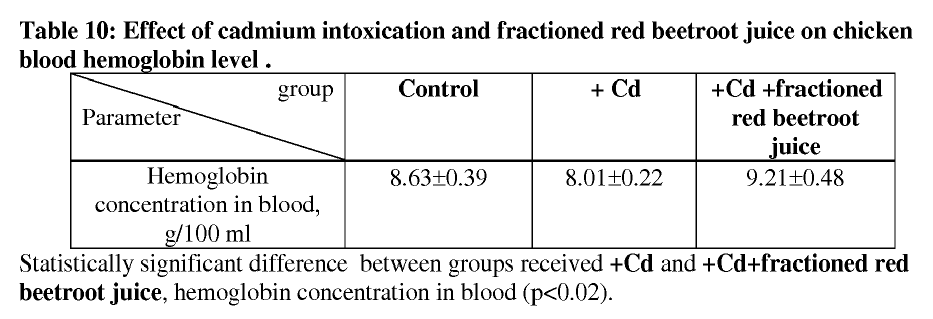

As the Table 10 shows the cadmium intoxication caused slight, however statistically significant decrease in blood hemoglobin level because the exposure time to cadmium was short, 10 days only. While preventive administration of fractioned red beetroot juice ensured the hemoglobin level increase.

Consumers' compliance to the fractioned red beetroot juice should be stressed. It is well known that fresh red beetroot juice before use should be kept for 3-5 hours in order to reduce undesirable side effects, e.g., flatulent stomach, nausea and other unpleasant reactions. Many potential users refuse to use the juice because of specific taste and smell. Fractioned juice is free from such specific features: it is supported by results of checkup of intestine condition in rats as well as organoleptic evaluation of the product. Fractioned red beetroot juice retains its natural color, it does not have the specific taste and smell, the product is sweetish and has a good taste when citric acid is added.

The results of these studies show that the red beetroot *Beta vulgaris* fractioned juice may be used in production of medicines for stimulating of iron absorption as well as in combination with substances for increasing iron level in humans and animals and for facilitation of hematopoiesis. Further the components of red beetroot juice preferably the fractioned juice may be used for production of food supplements and functional food stimulating iron ions absorption and hematopoiesis.

Based on the results of the studies the composition for facilitation of iron ions absorption and hematopoiesis obtained from red beetroot *(Beta vulgaris)* juice, preferably freshly extracted, containing complex of biologically active substances with upper limit of molecular weight up to 10 000 Da, preferably up to 5000 Da, is provided. The composition may be obtained from *Beta vulgaris* juice by gel-filtration, ultrafiltration or gel-electrophoresis with optional heating of the juice before filtration. According to one embodiment of the invention the composition is obtained by sequential filtrations of freshly extracted red beetroot (*Beta vulgaris*) juice through filters having pore size of 250 and 50 µm, heating for 5-15 minutes (preferably 10 minutes) at 80-100°C (preferably at 90°C), filtering through filters having pore size 250, 50, 5 and 1 µm followed by ultrafiltration through two filters: (i) capillary composite membrane (e.g., poly-(aryl-ether-sulfonic), polyvinylpirrolidone and polyamide) having ultrafiltration coefficient 70 mL/h*mmHg; (ii) capillary composite membrane (e.g., poly-(aryl-ether-sulfonic), polyvinylpirrolidone and polyamide) having ultrafiltration coefficient 10 mL/h*mmHg.

For further increase of specific biological activity of the fractioned red beetroot juice double concentration with drying in vacuum (from 665 to 2600 Pa) may be applied at 35-45°C, preferably at 40°C. These steps of the technology would be dependent on economic aspects of energy consumption by the technology.

The iron absorption and hematopoiesis stimulating composition additionally may contain at least one iron compound. Furthermore the composition may additionally contain known agents or compositions facilitating absorption of iron such as organic acids (including ascorbic acid, citric acid). Further aspect of the invention provides for the composition containing fractioned red beetroot juice together with 10-30 mg/100 ml bivalent iron (preferably 20 mg/100 ml), 300-380 mg/100 ml citric acid (preferably 320 mg/100 ml) and 25-100 mg/100 ml (preferably 50 mg/100 ml) ascorbic acid. The desirable concentration of preservatives is 100 mg/100 ml sodium benzoate and 50 mg/100 ml potassium sorbate which prevents disintegration of the end product during storage.

## Claims

1. A composition for stimulation of iron absorption and hematopoiesis obtainable from red beetroot *(Beta vulgaris)* juice, preferably freshly extracted, by isolation of a complex of biologically active substances having molecular weight up to 10 000 Da, preferably up to 5000 Da.

2. The composition according to claim 1, wherein the isolation of the complex of biologically active substances from the juice of *Beta vulgaris* is being performed by gel-filtration or ultrafiltration or gel-electrophoresis optionally with heating the juice of *Beta vulgaris* before the filtration.

3. The composition according to claim 1, wherein the isolation of the complex of biologically active substances from the juice of *Beta vulgaris* is being performed by filtration comprising the following steps:
(i) filtration of the said juice through filters having pore size 250 µm un 50 µm;
(ii) heating of the filtrate for 10-15 minutes at 80-100°C;
(iii) further filtration of the filtrate through the filters having pore size 250 µm, 50 µm, 5 µm and 1 µm.

4. The composition according to claim 1, wherein the isolation of the complex of biologically active substances from the juice of *Beta vulgaris* is being performed by ultrafiltration at 18-23°C in two sequential steps through a capillary composite membrane having ultrafiltration coefficient of 70 ml/h*mmHg followed by ultrafiltration through capillary composite filter having ultrafiltration coefficient of 10 ml/h*mmHg.

5. The composition according to any of the preceding claims, wherein the complex of biologically active substances is being concentrated two times by 665 to 2600 Pa vacuum drying at 35-45°C.

6. The composition according to any of the preceding claims, which additionally comprises at least one compound of bivalent iron in concentration of 10-30 mg/100 ml, preferably 20 mg/100 ml.

7. The composition according to any of the preceding claims which additionally comprises at least one agent or composition facilitating absorption of iron, such as organic acids or plant components.

8. The composition according to any of the preceding claims, which additionally comprises ascorbic acid in concentration of 25-100 mg/100 ml, preferably 50 mg/100 ml.

9. The composition according to any of the preceding claims, which additionally comprises citric acid in concentration of 300-380 mg/100 ml, preferably 320 mg/100 ml.

10. The composition according to any of the preceding claims, which additionally comprises sodium benzoate preferably in concentration of 100 mg/100 ml and potassium sorbate preferably in concentration of 50 mg/100 ml.

11. The composition according to any of the preceding claims, which is used for production of medicine for prevention of iron deficiency and/or treatment of iron deficiency anemia in subjects with alimentary iron deficiency or iron absorption disturbances, vaginal or digestive tract bleeding or during pregnancy.

12. The composition according to any of the preceding claims for use as a functional food or for production of food supplements.
